# EUROPEAN PATENT SPECIFICATION

(11) **EP 3 881 224 B1**
(45) Date of publication and mention of the grant of the patent: **15.04.2026**
(21) Application number: 19809196.9
(22) Date of filing: 08.11.2019
(51) Int. Cl.: G01N 15/14, G01N 15/10, G06F 18/23, G06V 10/762, G06V 20/69

(54) **INFORMATION PROCESSING APPARATUS, INFORMATION PROCESSING METHOD, AND PROGRAM**
INFORMATIONSVERARBEITUNGSVORRICHTUNG, INFORMATIONSVERARBEITUNGSVERFAHREN UND PROGRAMM
APPAREIL DE TRAITEMENT D'INFORMATIONS, PROCÉDÉ DE TRAITEMENT D'INFORMATIONS ET PROGRAMME

(30) Priority: 20.12.2018 US 201862782688 P; 18.06.2019 US 201916445075
(43) Date of publication of application: 22.09.2021
(73) Proprietor: Sony Group Corporation, Minato-ku, Tokyo 108-0075 (JP)
(72) Inventor: YAMANE, Kenji, Tokyo 108-0075 (JP); ENOKI, Junichiro, Tokyo 108-0075 (JP); MURATA, Rei, Tokyo 108-0075 (JP); FUTAMURA, Koji, Tokyo 108-0075 (JP); VELTRI, Gregory, New York, New York 10010 (US)
(74) Representative: D Young & Co LLP
(86) International application number: PCT/JP2019/043812
(87) International publication number: WO 2020/129454

(56) References cited:
- KIERAN O'NEILL ET AL: "Flow Cytometry Bioinformatics", PLOS COMPUTATIONAL BIOLOGY, vol. 9, no. 12, 5 December 2013 (2013-12-05), XP055665872, DOI: 10.1371/journal.pcbi.1003365
- Y. GE ET AL: "flowPeaks: a fast unsupervised clustering for flow cytometry data via K-means and density peak finding", BIOINFORMATICS, vol. 28, no. 15, 1 August 2012 (2012-08-01), pages 2052 - 2058, XP055073112, ISSN: 1367-4803, DOI: 10.1093/bioinformatics/bts300
- DANIEL JIMENEZ-CARRETERO ET AL: "Flow Cytometry Data Preparation Guidelines for Improved Automated Phenotypic Analysis", THE JOURNAL OF IMMUNOLOGY, vol. 200, no. 10, 7 May 2018 (2018-05-07), pages 3319 - 3331, XP055666227, ISSN: 0022-1767, DOI: 10.4049/jimmunol.1800446

## Description

### Technical Field

The present disclosure relates to an information processing apparatus, an information processing method, and a program.

### Background Art

The present disclosure relates to an information processing apparatus, an information processing method, and a program.

In fields such as medicine and biochemistry, to measure the properties of large numbers of cells rapidly, the use of a flow cytometer is becoming commonplace. A flow cytometer is an apparatus that optically measures the properties of cells by irradiating cells flowing through a flow cell with light, and detecting fluorescence emitted from the cells, scattered light, and the like.

Recently, for flow cytometers, the number of fluorescences that can be measured at one time is increasing. With this arrangement, since the increase in the dimensionality of the measurement data causes a combinatorial explosion to occur, it is becoming difficult to manually analyze the data measured with a flow cytometer.

For this reason, the analysis of multidimensional data measured with a flow cytometer by clustering through machine learning is being investigated, as disclosed in El-ad David Amir, et al, "viSNE enables visualization of high dimensional single-cell data and reveals phenotypic heterogeneity of leukemia", Nature Biotechnology, 2013 Jun, 31(6), 545-552.

However, in the case in which the amount of noise is the same in each dimension, the clustering performance drops for data of high dimensionality. For this reason, in the case of clustering the measurement data of a flow cytometer, it is typical to reduce the dimensionality by performing fluorescence separation and the like on the measurement data, and cluster the dimensionally-compressed data.

### Citation List

### Non Patent Literature

NPL 1: El-ad David Amir, et al, "viSNE enables visualization of high dimensional single-cell data and reveals phenotypic heterogeneity of leukemia", Nature Biotechnology, 2013 Jun, 31(6), 545-552

Further relevant prior art:
Kieran O'neill, et al, "Flow Cytometry Bioinformatics" PLos Computational Biology, 5 December 2013.

### Summary

However, in the case of performing dimension compression on multidimensional data, part of the information included in the measurement data is lost due to the dimension compression. For this reason, for example, in the case in which the clustering result of the multidimensional data is not appropriate, it is difficult for a user to go back to the measurement data of the flow cytometer and verify the validity of the clustering result.

Accordingly, the present disclosure proposes a novel and improved information processing apparatus, information processing method, and program capable of clustering measurement data using dimensionally-compressed data, while also enabling verification of the clustering result going back to the measurement data.
Embodiments of the present disclosures are defined by the appended claims.

According to an embodiment of the present disclosure as described above, it is possible to cluster measurement data using dimensionally-compressed data, while also enabling verification of the clustering result going back to the measurement data.

Note that the effects described above are not necessarily limitative. With or in the place of the above effects, there may be achieved any one of the effects described in this specification or other effects that may be grasped from this specification.

### Brief Description of Drawings

[fig.1]FIG. 1 is a schematic depiction that diagrammatically illustrates an exemplary configuration of a system including the information processing apparatus according to one embodiment of the present disclosure.
[fig.2]FIG. 2 is a block diagram illustrating an exemplary configuration of the information processing apparatus according to the embodiment.
[fig.3A]FIG. 3A is an explanatory diagram explaining a first detection mechanism of a measurement apparatus.
[fig.3B]FIG. 3B is an explanatory diagram explaining a second detection mechanism of a measurement apparatus.
[fig.4A]FIG. 4A is an explanatory diagram explaining a method of correcting fluorescence spillover for each wavelength band, and deriving the expression level of each fluorescent substance.
[fig.4B]FIG. 4B is an explanatory diagram explaining a method of correcting fluorescence spillover for each wavelength band, and deriving the expression level of each fluorescent substance.
[fig.5A]FIG. 5A is an explanatory diagram explaining a method of deriving the expression level of each fluorescent substance from an optical spectrum of fluorescent light.
[fig.5B]FIG. 5B is an explanatory diagram explaining a method of deriving the expression level of each fluorescent substance from an optical spectrum of fluorescent light.
[fig.6]FIG. 6 is an explanatory diagram illustrating one example of data stored by an information storage unit.
[fig.7A]FIG. 7A is an explanatory diagram illustrating one example of an image display that indicates a result of clustering by the information processing apparatus;
[fig.7B]FIG. 7B is an explanatory diagram illustrating one example of an image display that indicates a result of clustering by the information processing apparatus;
[fig.8A]FIG. 8A is an explanatory diagram illustrating one example of an image display that indicates first data, namely fluorescence-related information;
[fig.8B]FIG. 8B is an explanatory diagram illustrating one example of an image display that indicates second data, namely information related to the expression level of each fluorescent substance;
[fig.9]FIG. 9 is a flowchart illustrating an example of operations of the information processing apparatus according to the embodiment;
[fig.10]FIG. 10 is a block diagram schematically illustrating an exemplary configuration of an information processing apparatus according to a first modification;
[fig.11] FIG. 11 is an explanatory diagram explaining an overview of operations of the information processing apparatus according to the first modification;
[fig.12] FIG. 12 is a flowchart illustrating an example of operations of the information processing apparatus according to the first modification;
[fig.13] FIG. 13 is a flowchart illustrating another example of operations of the information processing apparatus according to the first modification;
[fig.14] FIG. 14 is a block diagram schematically illustrating an exemplary configuration of an information processing apparatus according to a second modification;
[fig.15]FIG. 15 is an explanatory diagram explaining an overview of operations of the information processing apparatus according to the second modification;
[fig.16]FIG. 16 is a flowchart illustrating an example of operations of the information processing apparatus according to the second modification; and
[fig.17]FIG. 17 is a block diagram illustrating an exemplary hardware configuration of the information processing apparatus according to the embodiment.

### Description of Embodiments

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.
1. Exemplary configuration of overall system
2. Exemplary configuration of information processing apparatus
3. Example of operations of information processing apparatus
4. Modifications
   4.1. First modification
   4.2. Second modification
5. Exemplary hardware configuration

### <1. Exemplary configuration of overall system>

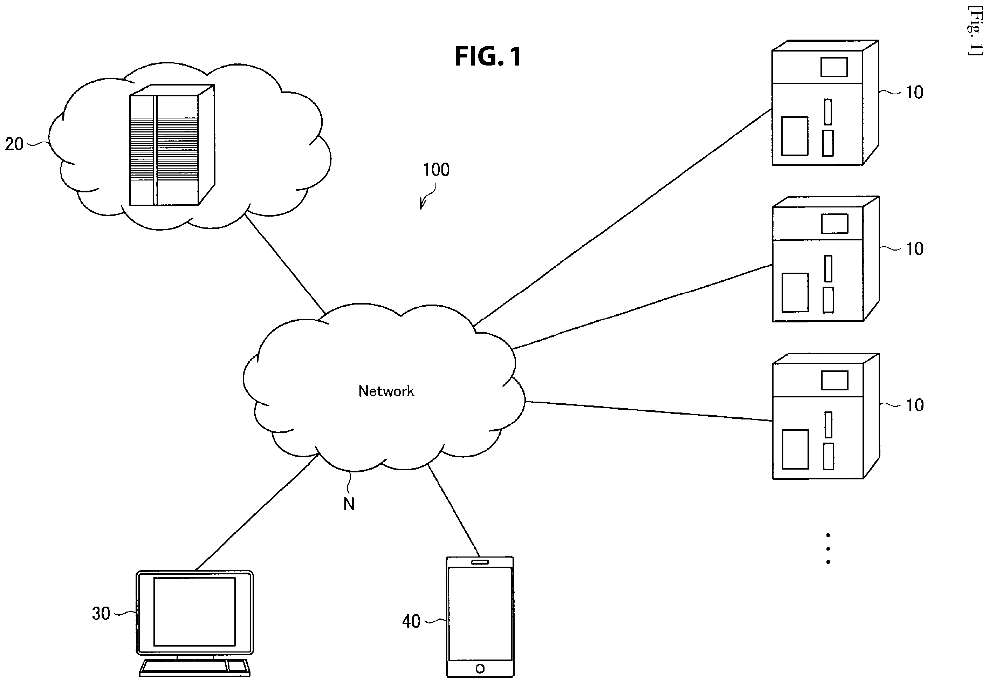

First, FIG. 1 will be referenced to describe a configuration of a system 100 including the information processing apparatus according to one embodiment of the present disclosure. FIG. 1 is a schematic depiction that diagrammatically illustrates an exemplary configuration of the system 100 including the information processing apparatus according to the present embodiment.

As illustrated in FIG. 1, the system 100 according to the present embodiment is provided with a measurement apparatus 10, an information processing apparatus 20, and terminal apparatus 30 and 40. The measurement apparatus 10, the information processing apparatus 20, and the terminal apparatus 30 and 40 are communicably interconnected through a network N. The network N may be, for example, an information communication network such as a mobile communication network, the Internet, or a local area network, or a combination of these multiple types of networks.

The measurement apparatus 10 is a measurement apparatus capable of detecting the fluorescence of each color from cells or the like targeted for measurement. The measurement apparatus 10 may be, for example, a flow cytometer that causes fluorescently stained cells to flow through a flow cell at high speed, and by irradiating the flowing cells with light rays, detects the fluorescence of each color of light from the cells.

The information processing apparatus 20 clusters each of the cells targeted for measurement on the basis of information related to the fluorescence of the cells measured by the measurement apparatus 10. With this arrangement, the information processing apparatus 20 is able to divide each of the cells measured by the measurement apparatus 10 into multiple groups (that is, clusters). Also, the information processing apparatus 20 stores the measurement data measured by the measurement apparatus 10 in association with clustering data obtained by performing dimension compression and the like to make the measurement data suitable for clustering. With this arrangement, the information processing apparatus 20 is able to reference the measurement data from before the dimension compression when judging the validity of the analysis result, while also reducing the time and cost taken for clustering by performing dimension compression and the like on the measurement data. For example, the information processing apparatus 20 may be a server or the like that is capable of processing large amounts of data quickly.

The terminal apparatus 30 and 40 are display apparatus or the like on which a clustering result from the information processing apparatus 20 is output, for example. For example, each of the terminal apparatus 30 and 40 may be a computer, a laptop, a smartphone, a tablet, or the like provided with a display unit that displays an analysis result received from the information processing apparatus 20 with an image, text, or the like.

In the system 100 including the information processing apparatus 20 according to the present embodiment, first, the information processing apparatus 20 acquires measurement data measured by the measurement apparatus 10 provided in each of a hospital, a clinic, or a laboratory via the network N. After that, the information processing apparatus 20 clusters the acquired measurement data, and outputs a clustering result to the terminal apparatus 30 and 40. Since clustering imposes a heavy information processing load, by intensively executing such processing on the information processing apparatus 20 that includes a dedicated server or the like, the efficiency of the system 100 as a whole may be improved. Furthermore, on the basis of a user selection, the information processing apparatus 20 controls the output of the measurement data and the clustering data obtained by processing the measurement data. With this arrangement, the information processing apparatus 20 is able to appropriately switch to and output information according to the user's demand.

Note that in the above, the measurement apparatus 10, the information processing apparatus 20, and the terminal apparatus 30 and 40 are described as being interconnected through the network N, but the technology according to the present disclosure is not limited to such an example. For example, the measurement apparatus 10, the information processing apparatus 20, and the terminal apparatus 30 and 40 may also be connected directly.

### <2. Exemplary configuration of information processing apparatus>

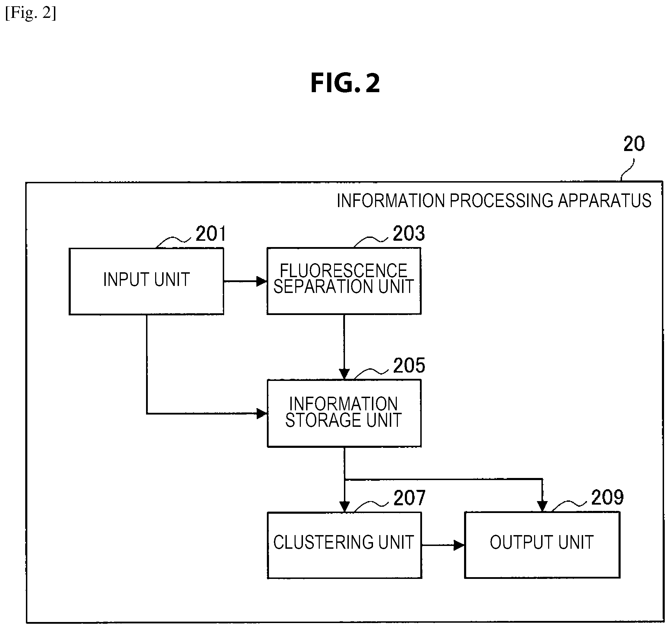

Next, FIG. 2 will be referenced to describe an exemplary configuration of the information processing apparatus 20 according to the present embodiment. FIG. 2 is a block diagram illustrating an exemplary configuration of the information processing apparatus 20 according to the present embodiment.

As illustrated in FIG. 2, the information processing apparatus 20 is provided with an input unit 201, a fluorescence separation unit 203, an information storage unit 205, a clustering unit 207, and an output unit 209. Note that a part of the functions of the information processing apparatus 20 (for example, the function of the fluorescence separation unit 203 described later) may also be provided in the measurement apparatus 10.

The input unit 201 acquires a result of measuring cells targeted for measurement from the measurement apparatus 10. Specifically, the input unit 201 acquires information related to the fluorescence of the cells targeted for measurement from the measurement apparatus 10. The input unit 201 is provided with an external input interface including a connection port, a communication apparatus, or the like for acquiring information from the measurement apparatus 10 through the network N, for example.

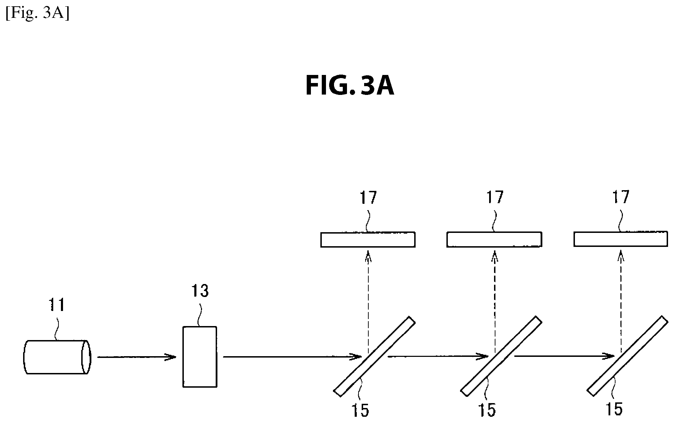

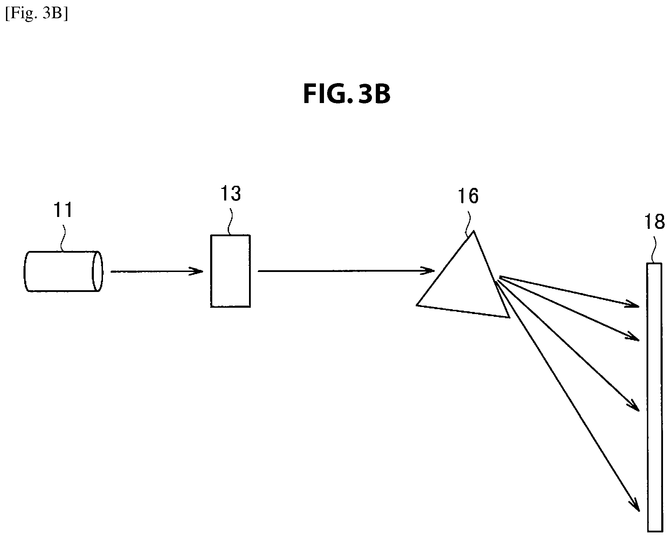

Herein, the content of the fluorescence-related information acquired by the input unit 201 is different depending on the fluorescence detection mechanism in the measurement apparatus 10. The specific content of the fluorescence-related information will be described together with the fluorescence detection mechanism in the measurement apparatus 10 illustrated in FIGS. 3A and 3B. FIG. 3A is an explanatory diagram explaining a first detection mechanism of the measurement apparatus 10, while FIG. 3B is an explanatory diagram explaining a second detection mechanism of the measurement apparatus 10.

As illustrated in FIG. 3A, in the first detection mechanism, by using dichroic mirrors 15 to spectrally separate fluorescent light obtained by irradiating a sample 13 with light rays from a light source 11, the intensity of the fluorescent light is measured for each of predetermined wavelength bands by photodetectors 17.

The dichroic mirrors 15 are mirrors that reflect light of a specific wavelength band while transmitting light of another wavelength band. The photodetectors 17 are photomultiplier tubes, photodiodes, or the like, for example. In the first measurement method, by providing the dichroic mirrors 15 that reflect light of different wavelength bands on the optical path of the fluorescent light from the sample 13, the fluorescent light from the sample 13 may be spectrally separated into each wavelength band. For example, in the first detection mechanism, by providing each of a dichroic mirror 15 that reflects light of the wavelength band corresponding to red, a dichroic mirror 15 that reflects light of the wavelength band corresponding to green, and a dichroic mirror 15 that reflects light of the wavelength band corresponding to blue in order from the side where light from the sample 13 is incident, the fluorescent light from the sample 13 may be spectrally separated into each wavelength band.

In the case in which the measurement apparatus 10 detects fluorescence with such a first detection mechanism, the fluorescence-related information acquired by the input unit 201 becomes information related to the intensity of the fluorescent light in each wavelength band.

Also, as illustrated in FIG. 3B, in the second detection mechanism, by using a prism 16 to spectrally separate fluorescent light obtained by irradiating the sample 13 with light rays from the light source 11, a continuous fluorescent spectrum is measured by a photodetector array 18.

The prism 16 is an optical member that disperses incident light, and the photodetector array 18 is a sensor in which multiple photodetectors (photomultipliers or photodiodes) are disposed in an array. In the second detection mechanism, by dispersing fluorescent light from the sample 13 with the prism 16 and detecting the dispersed light with the photodetector array 18, the fluorescent light from the sample 13 may be detected as a continuous spectrum.

In the case in which the measurement apparatus 10 detects fluorescence with such a second detection mechanism, the fluorescence-related information acquired by the input unit 201 becomes information related to the optical spectrum of the fluorescent light.

The fluorescence separation unit 203 separates each fluorescence included in the fluorescent light measured by the measurement apparatus 10, and thereby derives an expression level of a fluorescent substance corresponding to each fluorescence. The cells targeted for measurement are marked by multiple fluorescent substances, and the wavelength distributions of the fluorescence emitted from each of the fluorescent substances overlap each other. For this reason, by correcting the overlap of the wavelength distributions of the fluorescence emitted from each of the fluorescent substances and deriving net amount of light in each fluorescence, the fluorescence separation unit 203 is able to derive the expression level of each fluorescent substance and the expression level of biomolecules and the like marked by each fluorescent substance.

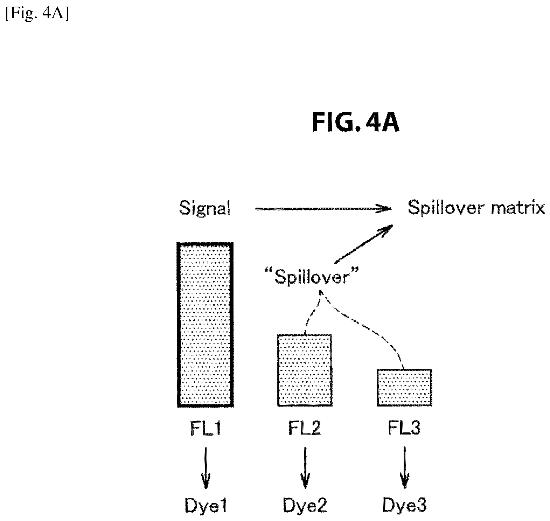

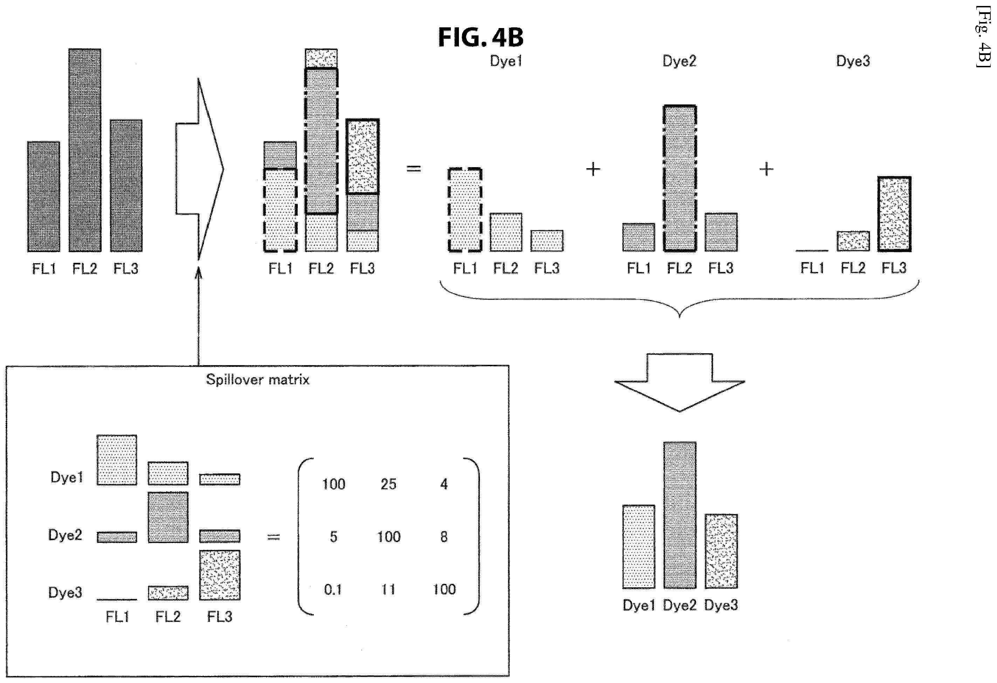

More specifically, in the case in which the fluorescence-related information acquired by the input unit 201 is information related to the intensity of the fluorescent light in each wavelength band, the fluorescence separation unit 203 is able to derive the expression level of each fluorescent substance by the method described with reference to FIGS. 4A and 4B. FIGS. 4A and 4B are explanatory diagrams explaining a method of correcting fluorescence spillover for each wavelength band, and deriving the expression level of each fluorescent substance.

As illustrated in FIG. 4A, in the case in which the fluorescence-related information is the intensity of the fluorescent light detected in each wavelength band, signals from photodetectors FL1, FL2, and FL3 that detect light for each wavelength band correspond to the fluorescence of fluorescent substances Dye1, Dye2, and Dye3. However, since the fluorescence from the fluorescent substances Dye1, Dye2, and Dye3 have a wavelength distribution, the signals detected by the photodetectors FL1, FL2, and FL3 also include fluorescent light spilled over from the other fluorescent substances.

For this reason, as illustrated in FIG. 4B, first, the fluorescence separation unit 203 acquires a spillover matrix indicating how much of the fluorescent light from the fluorescent substances Dye1, Dye2, and Dye3 spills over into each of the wavelength bands of the photodetectors FL1, FL2, and FL3. Next, on the basis of the spillover matrix, the fluorescence separation unit 203 separates the signals detected by the photodetectors FL1, FL2, and FL3 into the fluorescence from each of the fluorescent substances Dye1, Dye2, and Dye3. With this arrangement, the fluorescence separation unit 203 is able to derive the net amount of fluorescence from the fluorescent substances Dye1, Dye2, and Dye3, and thus is able to derive the expression levels of the fluorescent substances Dye1, Dye2, and Dye3.

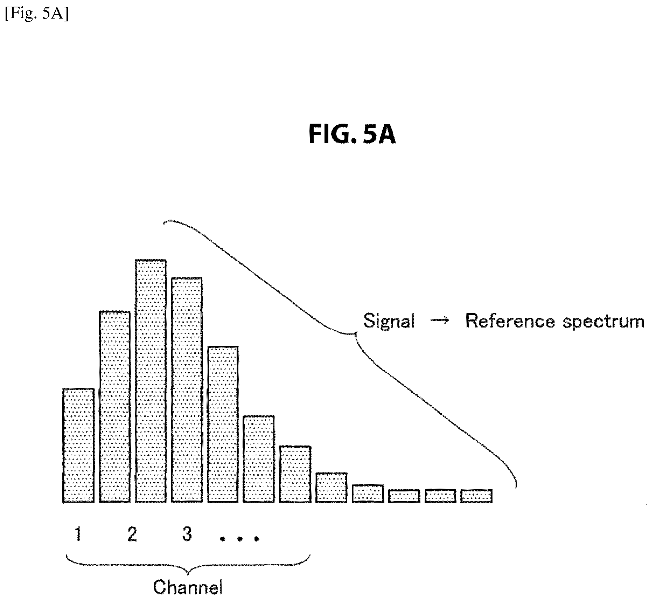

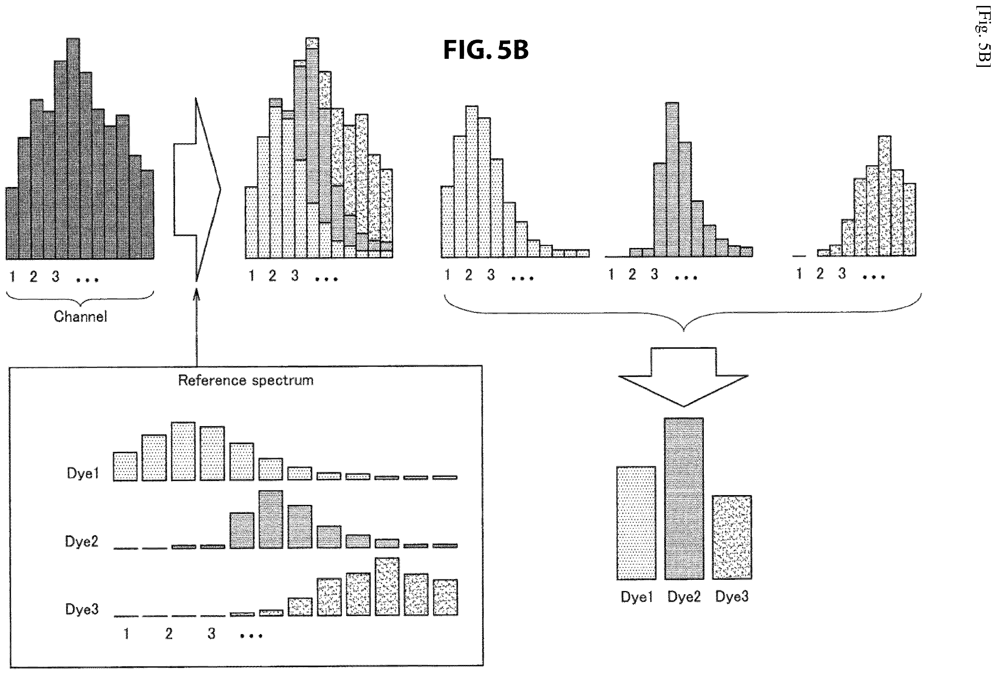

Also, in the case in which the fluorescence-related information acquired by the input unit 201 is information related to the optical spectrum of the fluorescent light, the fluorescence separation unit 203 is able to derive the expression level of each fluorescent substance by the method described with reference to FIGS. 5A and 5B. FIGS. 5A and 5B are explanatory diagrams explaining a method of deriving the expression level of each fluorescent substance from an optical spectrum of fluorescent light.

As illustrated in FIG. 5A, in the case in which the fluorescence-related information is the optical spectrum of the fluorescence, signals detected by multiple photodetectors Channel 1, 2, 3, and so on in the photodetector array are superpositions of the fluorescence from each of the fluorescent substances Dye1, Dye2, and Dye3.

For this reason, as illustrated in FIG. 5B, first, the fluorescence separation unit 203 acquires a reference spectrum for each of the fluorescent substances Dye1, Dye2, and Dye3 to detect. The reference spectrum indicates the optical spectrum of the fluorescence for each of the fluorescent substances Dye1, Dye2, and Dye3 individually. Next, by estimating the superposition of the reference spectrum of each of the fluorescent substances Dye1, Dye2, and Dye3 in the optical spectrum of the detected fluorescence, the fluorescence separation unit 203 is able to derive the expression level of each of the fluorescent substances Dye1, Dye2, and Dye3.

With this arrangement, even if the content of the fluorescence-related information acquired by the input unit 201 is any of the above, the fluorescence separation unit 203 is able to derive the expression level of the fluorescent substance corresponding to each fluorescence exhibited by the cells.

The information storage unit 205 stores the fluorescence-related information acquired by the input unit 201 in association with information related to the expression level of each fluorescent substance derived by the fluorescence separation unit 203. Specifically, the information storage unit 205 stores information related to fluorescence from cells obtained by the measurement apparatus 10 sensing a sample as first data, and stores information related to the expression level of each fluorescent substance obtained by separating the fluorescence from the cells into multiple fluorescences as second data. At this time, the information storage unit 205 stores this data as consolidated data by storing the first data and the second data derived from the first data in association with each other.

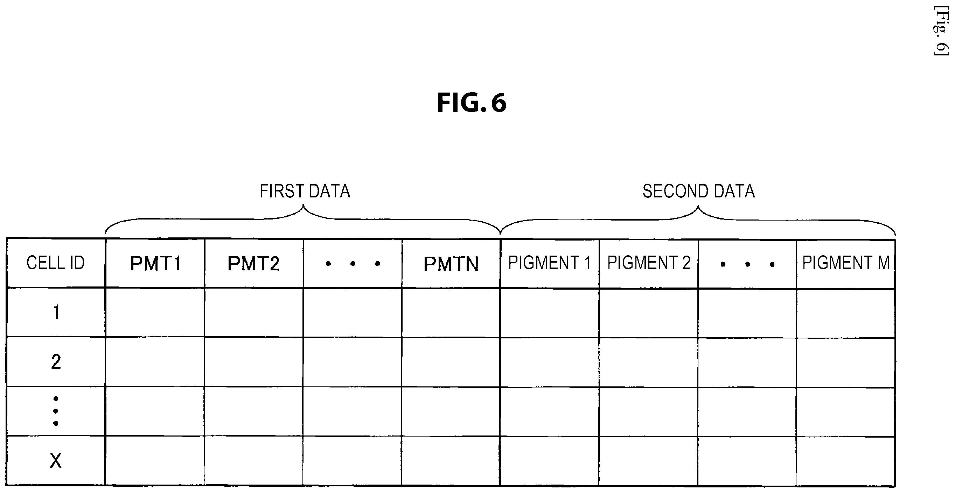

For example, the information storage unit 205 may store data consolidating the first data and the second data in a format as illustrated in FIG. 6. FIG. 6 is an explanatory diagram illustrating one example of data stored by the information storage unit 205.

As illustrated in FIG. 6, in the data stored by the information storage unit 205, identification information, namely a "Cell ID", is assigned to each cell. Also, as the first data, fluorescence intensities "PMT1" to "PMTN" detected by each of the photodetectors are stored for each cell. Additionally, as the second data, expression levels "Pigment 1" to "Pigment M" of each fluorescent substance are stored for each cell.

Herein, the first data is N-dimensional data including information about N fluorescence intensities "PMT1" to "PMTN", and the second data is M-dimensional data including information about M expression levels "Pigment 1" to "Pigment M". The dimensionality M of the second data is smaller than the dimensionality of the first data due to the fluorescence separation of the fluorescence separation unit 203.

At this point, since the efficiency and accuracy of clustering rises as the dimensionality becomes smaller, in the clustering unit 207 described later, cells are clustered using the second data. However, since the second data is dimensionally compressed in the fluorescence separation by the fluorescence separation unit 203, there is a possibility that information loss and the like may occur. For this reason, by storing both types of data in association with each other, the information storage unit 205 makes it possible to verify or confirm clustering going back to the measurement data of the measurement apparatus 10 easily, while also raising the efficiency and accuracy of the clustering.

Note that the first data and the second data stored in association with each other in the information storage unit 205 do not have to be information output from the input unit 201 and the fluorescence separation unit 203. For example, in the case in which the measurement apparatus 10 is provided with the fluorescence separation unit 203, the information processing apparatus 20 may acquire information related to the fluorescence of cells from the measurement apparatus 10 and information related to the expression level of each fluorescent substance in the cells, and the information storage unit 205 may store the acquired information in association with each other as the first data and the second data. Alternatively, the information processing apparatus 20 may acquire information related to the fluorescence of cells and information related to the expression level of each fluorescent substance in the cells stored in an external storage apparatus, and the information storage unit 205 may store the acquired information in association with each other as the first data and the second data.

The clustering unit 207 clusters cells on the basis of the expression level of each fluorescent substance in the cells derived by the fluorescence separation unit 203. In other words, the clustering unit 207 clusters cells on the basis of the second data stored by the information storage unit 205. Since the second data indicating the expression level of each fluorescent substance of the cells is multidimensional data, the information processing apparatus 20 is able to use clustering technology based on machine learning to divide the cells into multiple groups (clusters) faster than manually.

The clustering technique used by the clustering unit 207 is not particularly limited, and may be a publicly available clustering technique. For example, the clustering unit 207 may perform clustering using a typical clustering technique such as the ward method, the group average method, the single-link method, or the k-means method, or may also perform clustering using the self-organization map method.

The output unit 209 outputs the result of clustering by the clustering unit 207 to the terminal apparatus 30 and 40 or the like. For example, in the terminal apparatus 30 and 40, the output clustering result may be presented to a user as an image display.

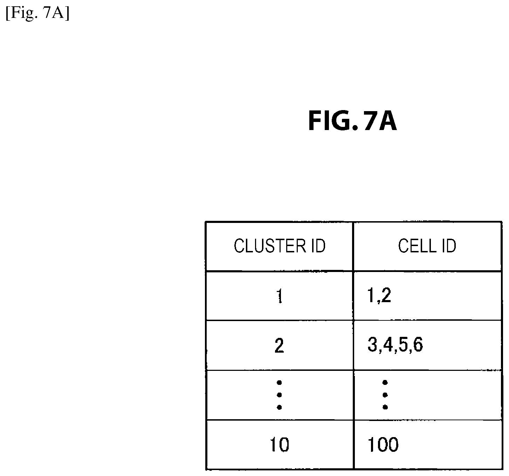

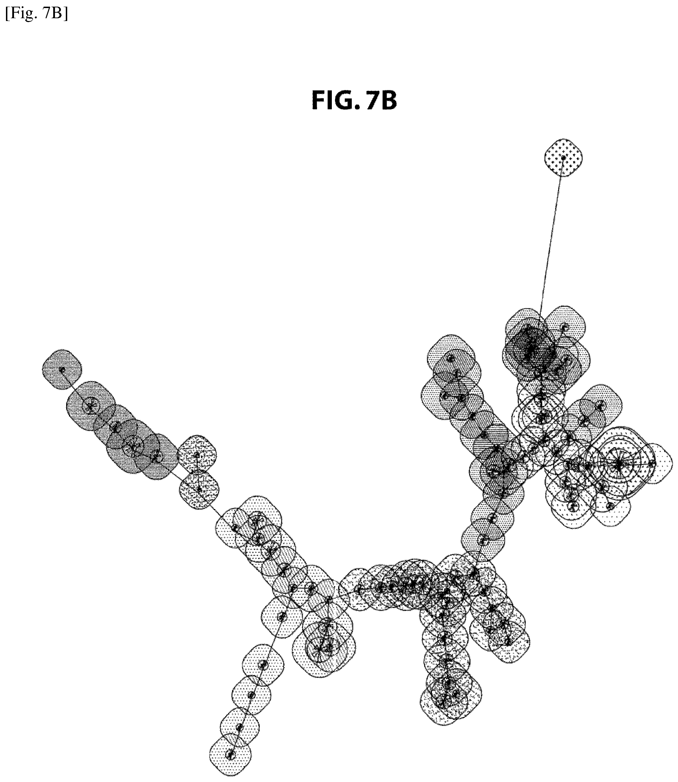

For example, the result of clustering by the clustering unit 207 may be displayed by the image displays illustrated in FIGS. 7A and 7B. FIGS. 7A and 7B are explanatory diagrams illustrating examples of image displays that indicate a result of clustering by the information processing apparatus 20.

For example, as illustrated in FIG. 7A, the result of clustering by the clustering unit 207 may be displayed by a display in a table format.

In the display illustrated in FIG. 7A, a group of 100 cells is divided into 10 clusters, and the cluster to which each cell belongs is indicated by identification numbers assigned to each cluster and each cell. Specifically, in the display illustrated in FIG. 7A, the cells with the identification numbers "1" and "2" belong to the cluster with the identification number "1", the cells with the identification numbers from "3" to "6" belong to the cluster with the identification number "2", and the cell with the identification number "100" belongs to the cluster with the identification number "10". According to such a display in a table format, how each cell belongs to each cluster may be indicated simply.

For example, as illustrated in FIG. 7B, the result of clustering by the clustering unit 207 may be displayed by a minimum spanning tree display.

In the display illustrated in FIG. 7B, radar charts differentiated by shading in multiple colors are arranged in an interconnected tree shape. Each radar chart illustrates each of the cells. Specifically, the distribution and size of each radar chart illustrates a vector corresponding to the expression level of each fluorescent substance in the cells. Herein, the regions different by shading in each color indicate each of the clusters to which each cell belongs. For example, cells indicated by the radar chart shaded in the same color (in FIG. 7B, the same hatching) illustrate that the cells belong to the same cluster.

Furthermore, in the display illustrated in FIG. 7B, the distance on the display between radar charts corresponds to the similarity between cells illustrated in the radar charts. In other words, cells illustrated by radar charts close to each other are similar to each other, whereas cells illustrated by radar charts distanced from each other are not similar to each other. According to such a minimum spanning tree display, in addition to how each cell belongs to each cluster, similarity relationships among the cells may be illustrated.

Also, for a cluster selected by the user, the output unit 209 additionally outputs data about the cells included in the cluster to the terminal apparatus 30 and 40 or the like. Specifically, the output unit 209 additionally outputs one or both of the first data and the second data to the terminal apparatus 30 and 40 or the like as data about the cells included in a cluster selected as a display target by the user. Whether the output unit 209 outputs the first data, the second data, or both the first data and the second data to the terminal apparatus 30 and 40 may be selected by the user, for example.

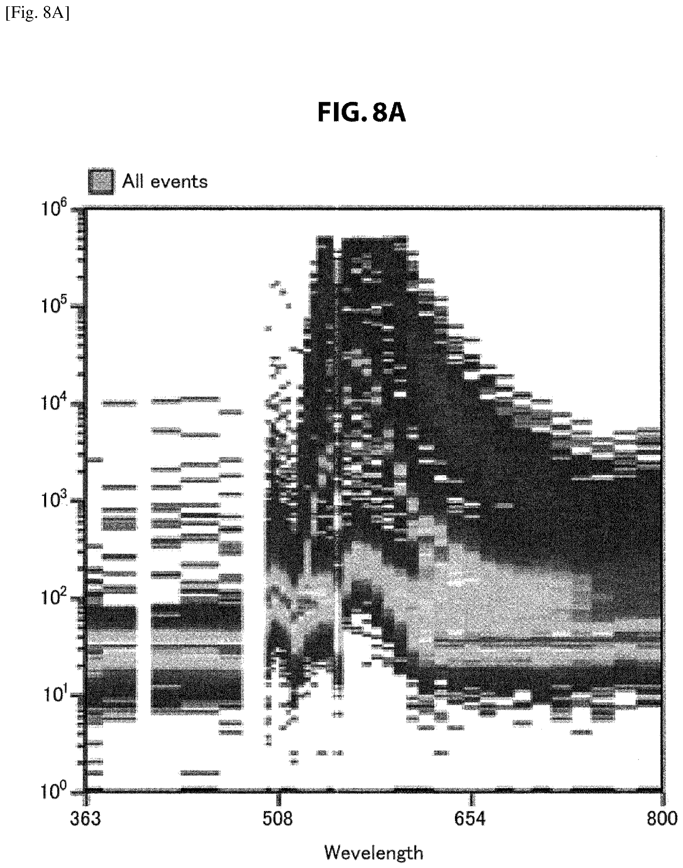

For example, the output unit 209 may output the first data to the terminal apparatus 30 and 40 as the image display illustrated in FIG. 8A. FIG. 8A is an explanatory diagram illustrating one example of an image display that indicates the first data, namely fluorescence-related information.

As illustrated in FIG. 8A, the output unit 209 may superpose the optical spectrum data of the fluorescence of each cell, and output an image display expressed as a bitmap to the terminal apparatus 30 and 40. By referring to the image display illustrated in FIG. 8A, the user is able to easily judge whether or not there is a malfunction in the measurement itself or the like.

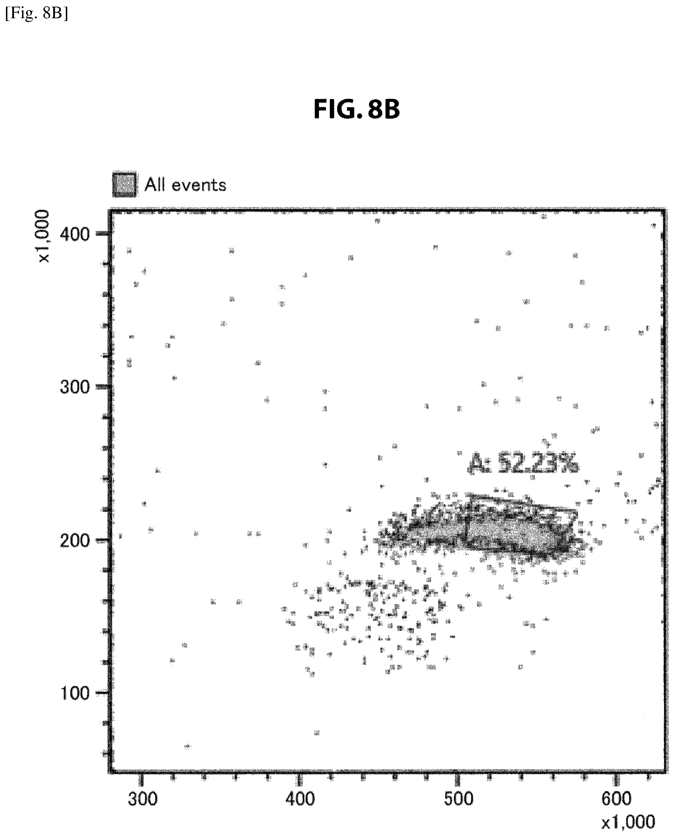

The output unit 209 may also output the second data to the terminal apparatus 30 and 40 as the image display illustrated in FIG. 8B. FIG. 8B is an explanatory diagram illustrating one example of an image display that indicates the second data, namely information related to the expression level of each fluorescent substance.

As illustrated in FIG. 8B, the output unit 209 may treat the expression levels of two fluorescent substances among each of the fluorescent substances of the cells as the vertical axis and the horizontal axis, and output an image display expressed as a scatter diagram to the terminal apparatus 30 and 40. By referring to the image display illustrated in FIG. 8B, the user is able to easily judge whether or not the clustering is valid or the like.

According to the information processing apparatus 20 provided with the above configuration, the user becomes able to reference information going back to the measurement data that has not undergone fluorescence separation or the like from the clustering result, and therefore is able to judge the reliability of the clustering and the reliability of the measurement result more easily. Consequently, the information processing apparatus 20 according to the present embodiment is able to improve the traceability of information with respect to the clustering result.

### <3. Example of operations of information processing apparatus>

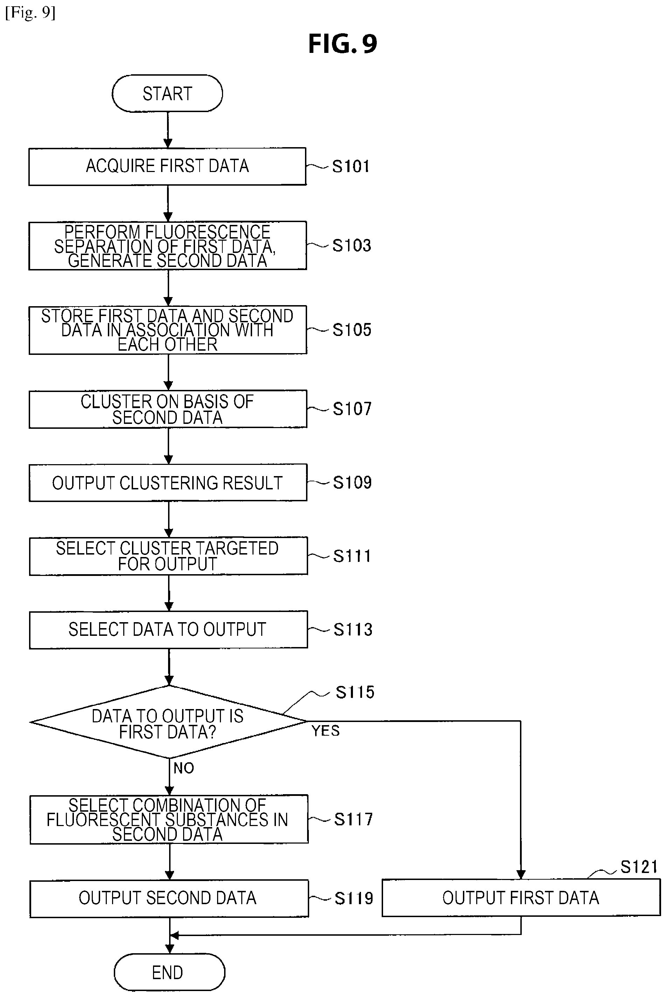

Next, FIG. 9 will be referenced to describe an exemplary configuration of the information processing apparatus 20 according to the present embodiment. FIG. 9 is a flowchart illustrating an example of operations of the information processing apparatus 20 according to the present embodiment.

As illustrated in FIG. 9, first, the input unit 201 acquires first data from the measurement apparatus 10 (S101). Specifically, the first data is information related to the fluorescence of cells targeted for measurement, and may be optical spectrum data of fluorescence from cells, for example. Next, by performing fluorescence separation on the first data, the fluorescence separation unit 203 generates second data (S103). Specifically, the second data is information related to the expression levels of fluorescent substances in cells, and the fluorescence separation unit 203 is able to generate the second data by separating each of the fluorescences from the optical spectrum of the first data.

Next, the information storage unit 205 stores the first data in association with the second data generated from the first data (S105). Next, the clustering unit 207 clusters the cells on the basis of the second data (S107). Specifically, the clustering unit 207 clusters the cells on the basis of the expression level of each fluorescent substance in the cells. The technique of the clustering by the clustering unit 207 is not particularly limited, and it is possible to use a publicly available technique.

After that, the output unit 209 outputs the result of clustering by the clustering unit 207 to the terminal apparatus 30 and 40 or the like (S109). At this time, assume that from the user who has checked the terminal apparatus 30 and 40 to which the clustering result has been output to an image display or the like, a cluster targeted for additional output is selected (S111), and which of the first data and the second data is to be output is selected (S113). With this arrangement, the output unit 209 confirms whether or not the data selected for output from the user is the first data (S113), and in the case in which the selected data is the first data (S113/Yes), the output unit 209 outputs the first data of each cell belonging to the selected cluster to the terminal apparatus 30 and 40 or the like (S121). On the other hand, in the case in which the selected data is the second data (S113/No), the output unit 209 causes the user to select a combination of fluorescent substances in the second data (S117), and outputs data about the expression levels of the selected combination of fluorescent substances from among the second data to the terminal apparatus 30 and 40 or the like (S119).

According to the above operations, the information processing apparatus 20 is able to go back to the first data and the second data from the clustering result and present information to the user. Consequently, the information processing apparatus 20 according to the present embodiment is able to improve the traceability of information with respect to the clustering result.

### <4. Modifications>

### (4.1. First modification)

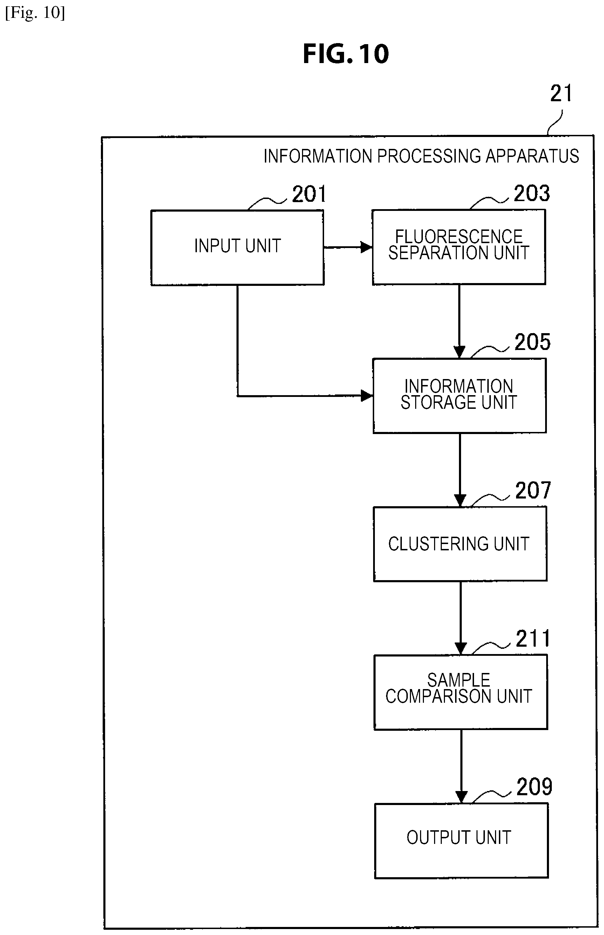

Next, FIGS. 10 to 13 will be referenced to describe a first modification of the information processing apparatus 20 according to the present embodiment. FIG. 10 is a block diagram schematically illustrating an exemplary configuration of an information processing apparatus 21 according to the first modification.

As illustrated in FIG. 10, the information processing apparatus 21 according to the first modification differs from the information processing apparatus 20 illustrated in FIG. 2 by additionally being provided with a sample comparison unit 211. In the following, the sample comparison unit 211 that is characteristic of the first modification will be described, while description will be omitted for the rest of the configuration that is substantially similar to the information processing apparatus 20 illustrated in FIG. 2.

The sample comparison unit 211 compares the clustering results of multiple samples, and specifies a cluster for which a disparity exists between the compared multiple samples. Specifically, in the case of comparing a first sample and a second sample, first, the sample comparison unit 211 maps each of the cells in the second sample onto the result of the clustering of the first sample by the clustering unit 207. Next, the sample comparison unit 211 compares the clustering result of the first sample to the mapping result of the second sample, and specifies a cluster in which the change between the clustering result of the first sample and the mapping result of the second sample is a threshold value or greater as a disparate cluster. The first data or the second data of the specified disparate cluster may be output to the terminal apparatus 30 and 40 by the output unit 209, for example. Note that, the first sample is a sample gathered from a healthy individual, for example, while the second sample is a sample gathered from a diseased individual, for example.

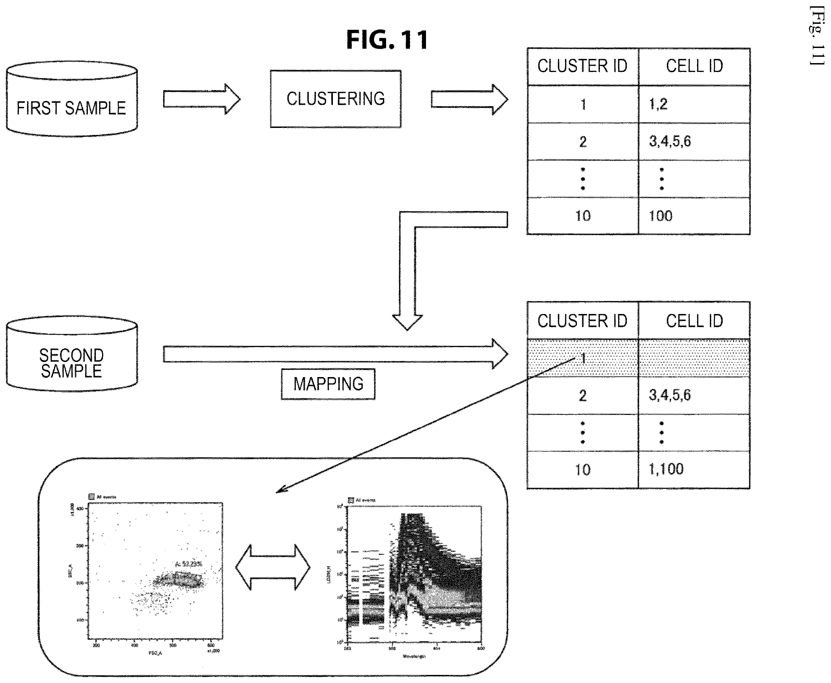

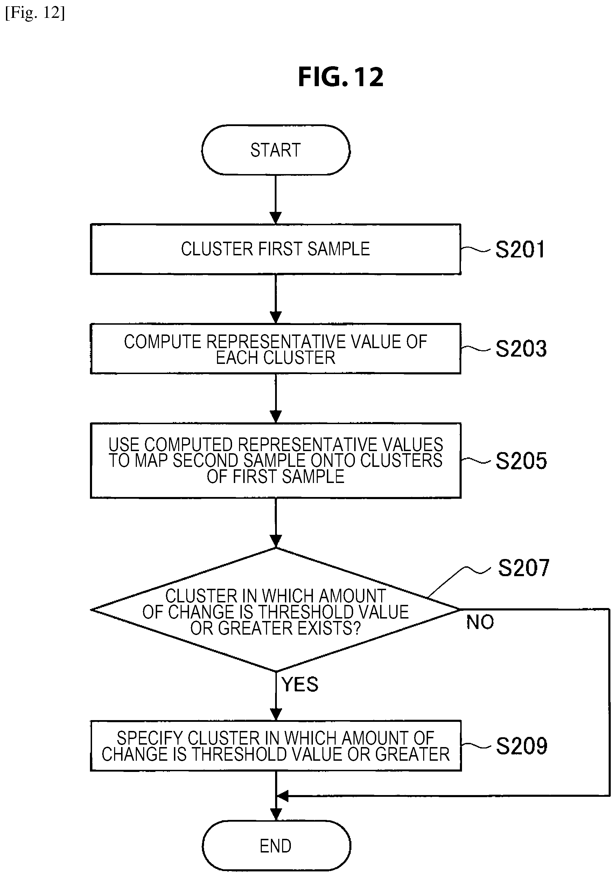

At this point, FIGS. 11 and 12 will be referenced to describe the operations of the sample comparison unit 211 more specifically. FIG. 11 is an explanatory diagram explaining an overview of operations of the information processing apparatus 21 according to the first modification. FIG. 12 is a flowchart illustrating an example of operations of the information processing apparatus 21 according to the first modification.

As illustrated in FIGS. 11 and 12, first, each first sample is clustered on the basis of the second data by the clustering unit 207 (S201).

Next, in each of the clusters clustering each first sample, the sample comparison unit 211 computes representative values of the second data of the cells belonging to each cluster (S203). For example, the sample comparison unit 211 may treat the mean, the mode, or the median of each expression level of each fluorescent substance in the second data as the representative values.

Next, on the basis of the second data of the second sample, the sample comparison unit 211 maps each cell of the second sample onto the cluster with the shortest distance among the clusters in the clustering result of the first sample (S205). Specifically, the sample comparison unit 211 calculates the Euclidean distance or the Manhattan distance between a vector of the second data of each cell of the second sample and the representative value of a cluster that is a clustering result of the first sample.

Next, the sample comparison unit 211 compares the clustering result of the first sample and the mapping result of the second sample, and determines the existence or non-existence of a cluster in which the number of belonging cells has changed by a threshold value or greater between the first sample and the second sample (S207). In the case in which a cluster in which the number of belonging cells has changed by the threshold value or greater between the first sample and the second sample does not exist (S207/No), the information processing apparatus 21 ends operation.

On the other hand, in the case in which a cluster in which the number of belonging cells has changed by the threshold value or greater between the first sample and the second sample exists (S207/Yes), the sample comparison unit 211 specifies the cluster as a disparate cluster (S209). For example, the sample comparison unit 211 may specify a cluster in which the number of belonging cells has changed by a threshold value (such as 2, for example) or greater between the clustering result of the first sample and the mapping result of the second sample as a disparate cluster. Alternatively, the sample comparison unit 211 may specify a cluster in which the proportion of the number of belonging cells with respect to the sample as a whole has changed by a threshold value or greater between the clustering result of the first sample and the mapping result of the second sample as a disparate cluster.

The first data and/or second data of the disparate cluster specified by the sample comparison unit 211 is output by the output unit 209 as an image display or the like to the terminal apparatus 30 and 40, and thereby presented to the user. With this arrangement, the user is able to check information related to the fluorescence and information related to the expression level of each fluorescent substance in a cell group for which a disparity exists between the first sample and the second sample.

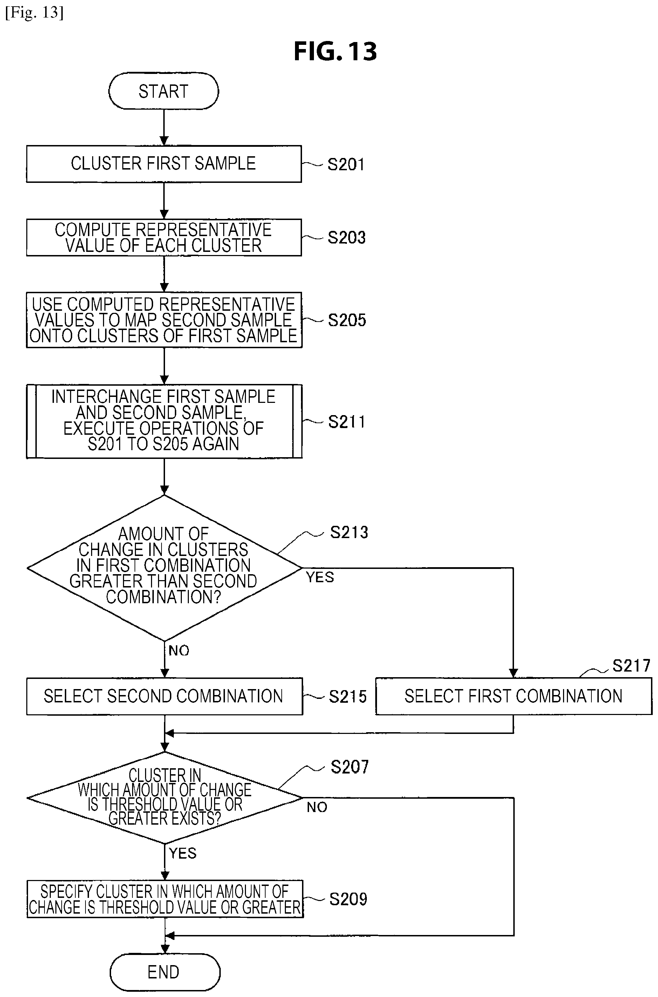

Next, FIG. 13 will be referenced to described another example of operations of the information processing apparatus 21 according to the first modification. FIG. 13 is a flowchart illustrating another example of operations of the information processing apparatus 21 according to the first modification.

In the example of operations according to the flowchart illustrated in FIG. 12, for example, in the case in which a cell group included in the second sample only exists, since the cell group does not form a cluster in the first sample, there is a possibility that the cell group will be mapped to the entire clustering result of the first sample. Accordingly, in the other example of operations illustrated in FIG. 13, the combination of the clustered sample and the mapped sample is interchanged, and each is clustered and mapped. With this arrangement, in the other example of operations illustrated in FIG. 13, even in the case in which a cell group included in only one of either the first sample or the second sample exists, it becomes possible to specify a cell group with a disparity between the first sample and the second sample.

As illustrated in FIG. 13, first, each first sample is clustered on the basis of the second data by the clustering unit 207 (S201). Next, in each of the clusters clustering each first sample, the sample comparison unit 211 computes representative values of the second data of the cells belonging to each cluster (S203). Next, on the basis of the second data of the second sample, the sample comparison unit 211 maps each cell of the second sample onto the cluster with the shortest distance among the clusters in the clustering result of the first sample (S205). Herein, the combination of the clustered sample (first sample) and the mapped sample (second sample) in S201 to S205 will also be designated the first combination.

Next, the relationships of clustering and mapping of the first sample and the second sample are interchanged, and the operations of S201 to S205 above are executed (S211).

Specifically, each second sample is clustered on the basis of the second data by the clustering unit 207. Next, in each of the clusters clustering each second sample, the sample comparison unit 211 computes representative values of the second data of the cells belonging to each cluster. Next, on the basis of the second data of the first sample, the sample comparison unit 211 maps each cell of the first sample onto the cluster with the shortest distance among the clusters in the clustering result of the second sample. Herein, the combination of the clustered sample (second sample) and the mapped sample (first sample) in S211 will also be designated the second combination.

Next, the sample comparison unit 211 determines whether or not the amount of change in each cluster for the case of mapping the second sample onto the clustering result of the first sample (the first combination) is greater than the amount of change in each cluster for the case of mapping the first sample onto the clustering result of the second sample (the second combination) (S213). In the case in which the amount of change in each cluster of the first combination is greater (S213/Yes), the sample comparison unit 211 selects the first combination (S217), whereas in the case in which the amount of change in each cluster of the second combination is greater (S213/No), the sample comparison unit 211 selects the second combination (S215). The comparison between the amount of change in each cluster of the first combination and the amount of change in each cluster of the second combination may be made according to the maximum value of the amounts of change for all clusters, or according to the number of clusters in which the amount of change is a threshold value or greater, for example.

After that, the sample comparison unit 211 compares the clustering result and the mapping result in the selected combination, and determines the existence or non-existence of a cluster in which the number of belonging cells has changed by a threshold value or greater between the clustering result and the mapping result (S207). In the case in which a cluster in which the number of belonging cells has changed by the threshold value or greater between the clustering result and the mapping result does not exist (S207/No), the information processing apparatus 21 ends operation.

On the other hand, in the case in which a cluster in which the number of belonging cells has changed by the threshold value or greater between the clustering result and the mapping result exists (S207/Yes), the sample comparison unit 211 specifies the cluster as a disparate cluster (S209).

The first data and/or second data of the disparate cluster specified by the sample comparison unit 211 is output by the output unit 209 as an image display or the like to the terminal apparatus 30 and 40, and thereby presented to the user. With this arrangement, the user is able to check information related to the fluorescence and information related to the expression level of each fluorescent substance in a cell group for which a disparity exists between the first sample and the second sample.

### (4.2. Second modification)

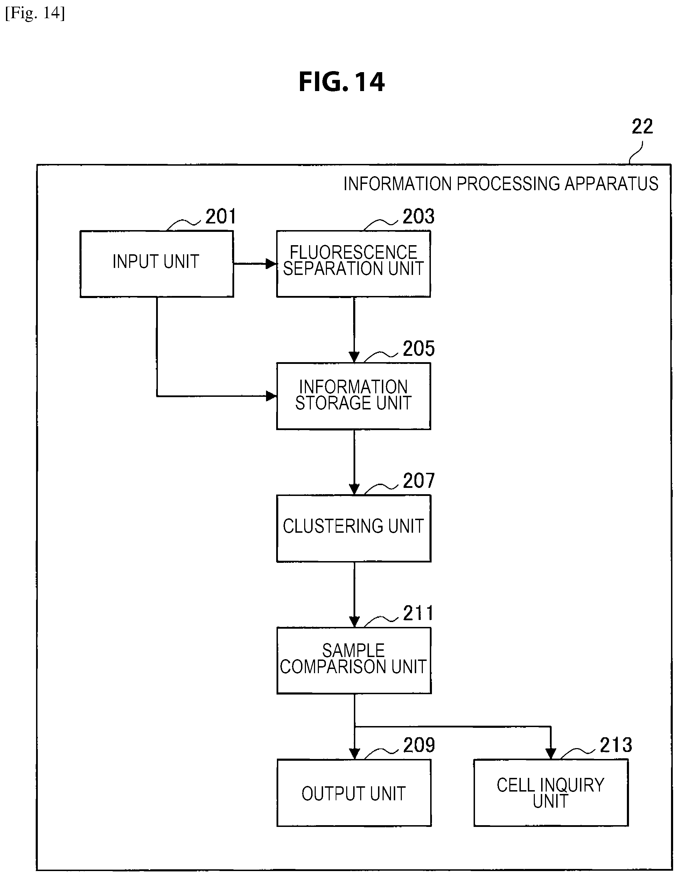

Next, FIGS. 14 to 16 will be referenced to describe a second modification of the information processing apparatus 20 according to the present embodiment. FIG. 14 is a block diagram schematically illustrating an exemplary configuration of an information processing apparatus 22 according to the second modification.

As illustrated in FIG. 14, the information processing apparatus 22 according to the second modification is provided with a cell inquiry unit 213 in addition to the information processing apparatus 21 according to the first modification. In the following, the cell inquiry unit 213 that is characteristic of the second modification will be described, while description will be omitted for the rest of the configuration that is substantially similar to the information processing apparatus 21 illustrated in FIG. 10.

The cell inquiry unit 213 queries an external database to specify which cell type the disparate cluster specified by the sample comparison unit 211 corresponds to biologically. Specifically, the cell inquiry unit 213 generates information related to an expression pattern of cells included in a disparate cluster from the second data of the disparate cluster specified by the sample comparison unit 211. Next, by inputting information related to the generated expression pattern into an external ontology database, the cell inquiry unit 213 specifies which cell group the disparate cluster corresponds to. Information about the specified cell group may be presented to the user by being output to the terminal apparatus 30 and 40 by the output unit 209, for example.

For the external ontology database, a public database such as the "cell ontology database (https://bioportal.bioontology.org/ontologies/CL)", for example, or the "flowCL (https://bioconductor.org/packages/release/bioc/html/flowCL.html)" database may be used.

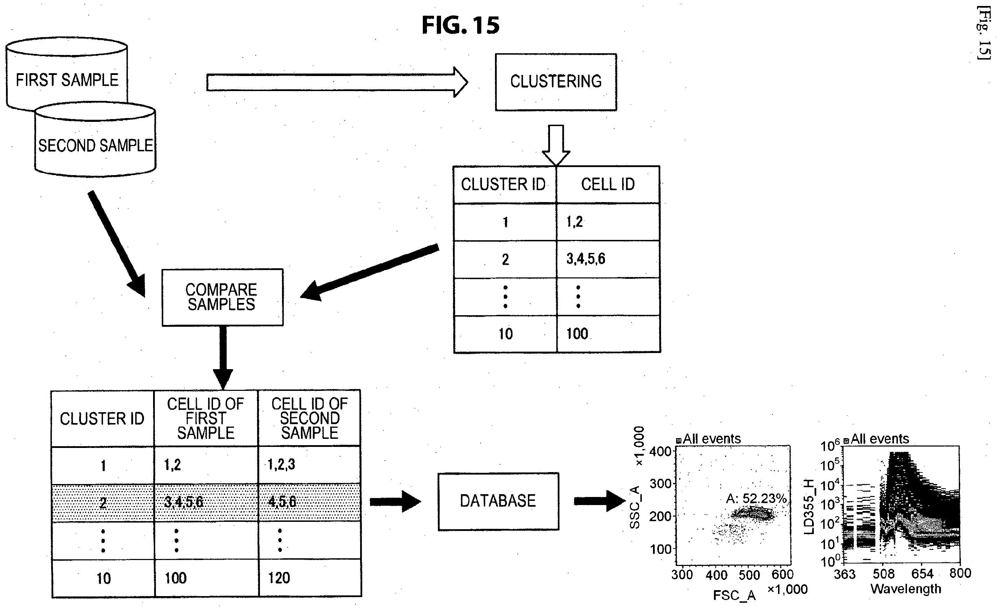

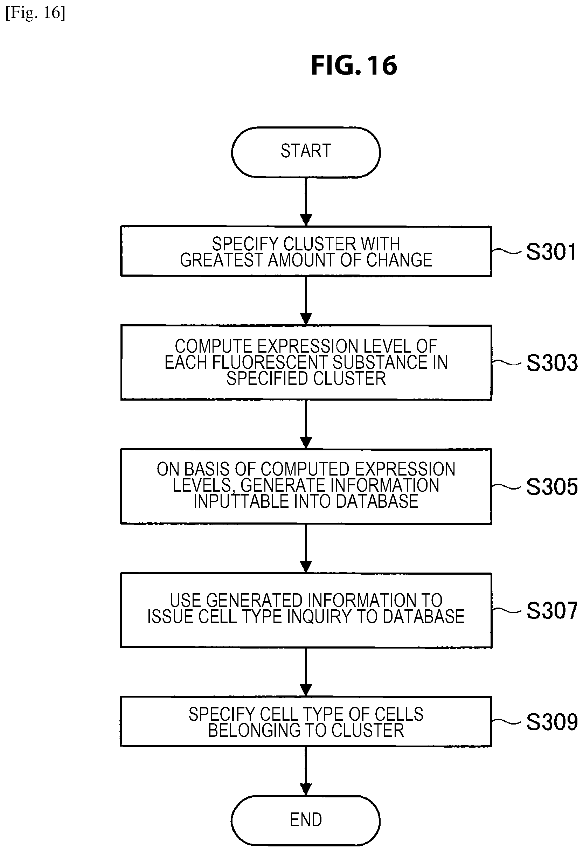

At this point, FIGS. 15 and 16 will be referenced to describe the operations of the cell inquiry unit 213 more specifically. FIG. 15 is an explanatory diagram explaining an overview of operations of the information processing apparatus 22 according to the second modification. FIG. 16 is a flowchart illustrating an example of operations of the information processing apparatus 22 according to the second modification.

As illustrated in FIGS. 15 and 16, first, as described in the example of operations of the information processing apparatus 21 according to the first modification, clustering and mapping of the first sample and the second sample are performed. Assume that by this arrangement, a disparate cluster for which a disparity exists between the first sample and the second sample is specified (S301).

At this point, the cell inquiry unit 213 computes a representative value of the expression level of each fluorescent substance in the disparate cluster from the second data of the cells included in the disparate cluster (S303). For example, the cell inquiry unit 213 may treat the mean, the mode, or the median of the expression level of each fluorescent substance in each cell included in the disparate cluster as the representative value of the expression level of each fluorescent substance in the disparate cluster.

Next, the cell inquiry unit 213 generates information that is inputtable into an external database, on the basis of the computed expression level of each fluorescent substance in the disparate cluster (S305). For example, in the case of using "flowCL" as the external database, the cell inquiry unit 213 may generate information (such as CD3+; CD8-; CD20+, for example) that specifies the positivity or negativity of expression of each marker molecule in the cells.

The positivity or negativity of expression of each marker molecule may be decided relatively by setting an appropriate threshold value such that the expression levels of fluorescent substances in all clusters are dichotomized, and determining whether or not the representative value of the expression level of each fluorescent substance in the disparate cluster exceeds the threshold value. Alternatively, the positivity or negativity of each marker molecule may be decided absolutely on the basis of whether or not the representative value of the expression level of each fluorescent substance in the disparate cluster exceeds a predesignated threshold value.

Next, by inputting the generated information into the external database, the cell inquiry unit 213 queries the cell type of the cells included in the disparate cluster (S307). After that, the cell inquiry unit 213 specifies the cell type of the cells belonging to the disparate cluster on the basis of the query result (S309).

The cell type of the disparate cluster specified by the cell inquiry unit 213 is output to the terminal apparatus 30 and 40 by the output unit 209, and presented to the user as an image display or the like. In addition, the output unit 209 may also output the first data or the second data of the disparate cluster to the terminal apparatus 30 and 40 as well. With this arrangement, the user is able to check the kind of biological cell type of a cell group for which a disparity exists between the first sample and the second sample.

### <5. Exemplary hardware configuration>

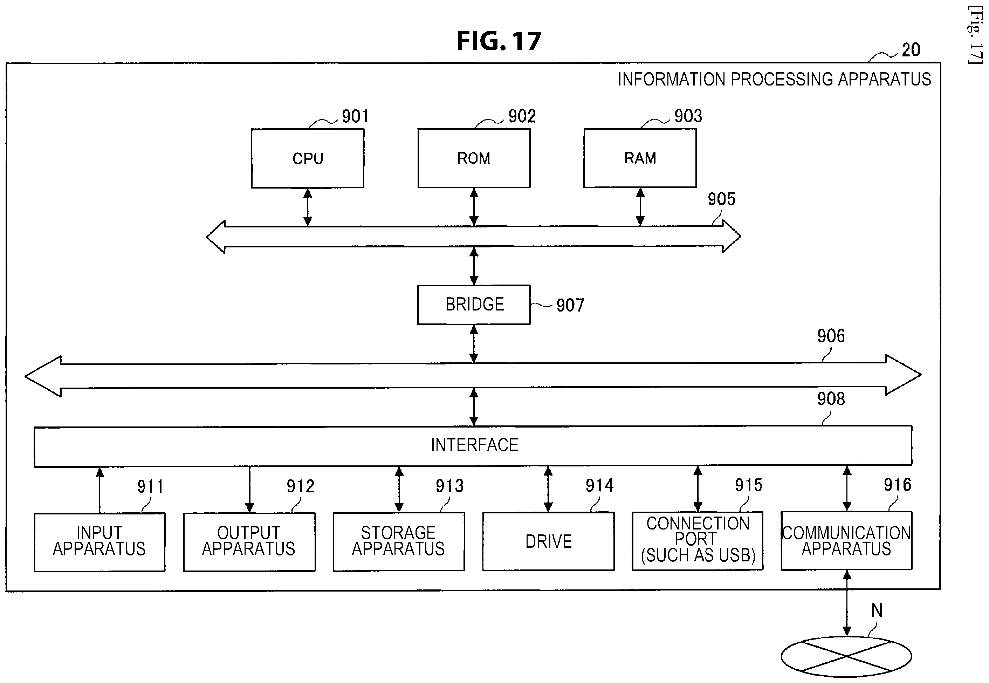

Next, FIG. 17 will be referenced to describe a hardware configuration of the information processing apparatus 20 according to the present embodiment. FIG. 17 is a block diagram illustrating an exemplary hardware configuration of the information processing apparatus 20 according to the present embodiment.

As illustrated in FIG. 17, the information processing apparatus 20 is provided with a central processing unit (CPU) 901, read-only memory (ROM) 902, random access memory (RAM) 903, a bridge 907, internal buses 905 and 906, an interface 908, an input apparatus 911, an output apparatus 912, a storage apparatus 913, a drive 914, a connection port 915, and a communication apparatus 916.

The CPU 901 functions as a computational processing device and control device, and controls the overall operation of the information processing apparatus 20 by following various programs stored in the ROM 902 and the like. The ROM 902 stores programs and computational parameters used by the CPU 901, while the RAM 903 stores programs used during execution by the CPU 901 and parameters that change appropriately during such execution. For example, the CPU 901 may execute the functions of the fluorescence separation unit 203, the clustering unit 207, the sample comparison unit 211, and the cell inquiry unit 213.

The CPU 901, the ROM 902, and the RAM 903 are interconnected by the bridge 907, the internal buses 905 and 906, and the like. In addition, the CPU 901, the ROM 902, and the RAM 903 are also connected to the input apparatus 911, the output apparatus 912, the storage apparatus 913, the drive 914, the connection port 915, and the communication apparatus 916 through the interface 908. For example, the RAM 903 may execute the functions of the information storage unit 205.

The input apparatus 911 includes input devices that accept the input of information, such as a touch panel, a keyboard, a mouse, a button, a microphone, a switch, or a lever. Additionally, the input apparatus 911 also includes an input control circuit or the like that generates an input signal on the basis of input information, and outputs the generated input signal to the CPU 901. The input apparatus 911 may execute the functions of the input unit 201, for example.

The output apparatus 912 includes a display device such as a cathode ray tube (CRT) display device, a liquid crystal display device, or an organic electroluminescence (EL) display device, for example. Additionally, the output apparatus 912 may also include an audio output device such as a speaker or headphones. The output apparatus 912 may execute the functions of the output unit 209, for example.

The storage apparatus 913 is a storage device used for data storage in the information processing apparatus 20. The storage apparatus 913 may include a storage medium, a storage device that stores data in the storage medium, a readout device that reads out data from the storage medium, and a deletion device that deletes data stored in the storage medium.

The drive 914 is a reader/writer for a storage medium, and is internally housed inside, or externally attached to, the information processing apparatus 20. For example, the drive 914 reads out information stored in a removable storage medium such as an inserted magnetic disk, optical disc, magneto-optical disc, or semiconductor memory, and outputs the information to the RAM 903. It is also possible for the drive 914 to write information to a removable storage medium.

The connection port 915 is a connection interface including connection ports for connecting with externally connected equipment, such as a Universal Serial Bus (USB) port, an Ethernet (registered trademark) port, an IEEE 802.11 standard port, and an optical audio terminal.

The communication apparatus 916 is a communication interface including a communication device or the like that connects to the network N, for example. Also, the communication apparatus 916 may be a communication apparatus supporting wired or wireless LAN, and may also be a cable communication apparatus that communicates over a wired cable. The communication apparatus 916 and the connection port 915 may execute the functions of the input unit 201 and the output unit 209, for example.

Note that it is also possible to create a computer program for causing hardware such as a CPU, ROM, and RAM built into the information processing apparatus 20 to exhibit functions similar to each component of the information processing apparatus according to the present embodiment described above. It is also possible to provide a storage medium having such a computer program stored therein.

It should be understood by those skilled in the art that various modifications, combinations, sub-combinations and alterations may occur depending on design requirements and other factors insofar as they are within the scope of the appended claims.

## Claims

1. An information processing apparatus (20, 21) comprising:
an information storage unit (205) that stores a result of sensing light from cells, namely first data, and a result of separating the first data into a plurality of fluorescences, namely second data, in association with each other;
a clustering unit (207) that clusters the cells into a plurality of clusters on a basis of the second data; and
an output unit (209) that outputs a clustering result from the clustering unit, wherein
the output unit additionally outputs at least one or more of the first data and the second data about the cells included in a cluster selected by a user from among the plurality of clusters;
a sample comparison unit (211) that compares a first sample comprising a plurality of cells, for which the first data and the second data are stored in the information storage unit, and a second sample comprising a plurality of cells to each other;
wherein
the sample comparison unit (211) is configured to compute, for each of the clusters of a clustering result of the first sample, a representative value of the second data of cells of the first sample belonging to each cluster and map on the basis of second data of the second sample each of the cells in the second sample onto the cluster with the shortest distance among clusters of the clustering result of the first sample;
wherein
the sample comparison unit compares the clustering result of the first sample and the mapping result of the second sample, and thereby specifies a cluster in which an amount of change between the first sample and the second sample is a threshold value or greater, and
the output unit additionally outputs at least one or more of the first data and the second data about the cells included in the specified cluster.

2. The information processing apparatus according to claim 1, wherein
a dimensionality of the first data is greater than a dimensionality of the second data.

3. The information processing apparatus according to claim 1, wherein
the first data is optical spectrum data of light from the cells.

4. The information processing apparatus according to claim 1, wherein
the second data is output as a combination of fluorescences selected from among the plurality of fluorescences.

5. The information processing apparatus according to claim 1, wherein
the clustering result is output as an image display.

6. The information processing apparatus according to claim 1, further comprising:
a cell inquiry unit that inputs information related to the cells included in the cluster specified by the sample comparison unit into a database for specifying the cells.

7. The information processing apparatus according to claim 6, wherein
the cell inquiry unit specifies a cell type of the cells included in the cluster on a basis of a result of inquiring the database, and
the output unit additionally outputs at least one or more of the first data and the second data about the cells with the specified cell type.

8. The information processing apparatus according to claim 6, wherein
the information related to the cells that is input into the database is generated on a basis of the second data.

9. An information processing method comprising:
storing a result of sensing light from cells, namely first data, and a result of separating the first data into a plurality of fluorescences, namely second data, in association with each other;
clustering the cells into a plurality of clusters on a basis of the second data; and
outputting a clustering result from the clustering unit; and
additionally outputting at least one or more of the first data and the second data about the cells included in a cluster selected by a user from among the plurality of clusters;
comparing a first sample comprising a plurality of cells, for which the first data and the second data are stored in the information storage unit, and a second sample comprising a plurality of cells to each other;
wherein the comparing comprises computing for each of the clusters of a clustering result of the first sample, a representative value of the second data of cells of the first sample belonging to each cluster and mapping on the basis of second data of the second sample each of the cells in the second sample onto the cluster with the shortest distance among clusters of the clustering result of the first sample;
wherein the method further comprises comparing the clustering result of the first sample and the mapping result of the second sample, and thereby specifies a cluster in which an amount of change between the first sample and the second sample is a threshold value or greater, and
outputting at least one or more of the first data and the second data about the cells included in the specified cluster.

10. A program causing a computer to function as:
an information storage unit (205) that stores a result of sensing light from cells, namely first data, and a result of separating the first data into a plurality of fluorescences, namely second data, in association with each other;
a clustering unit (207) that clusters the cells into a plurality of clusters on a basis of the second data; and
an output unit (209) that outputs a clustering result from the clustering unit, wherein
the output unit is made to function to additionally output at least one or more of the first data and the second data about the cells included in a cluster selected by a user from among the plurality of clusters;
a sample comparison unit (211) that compares a first sample comprising a plurality of cells, for which the first data and the second data are stored in the information storage unit, and a second sample comprising a plurality of cells to each other;
wherein
the sample comparison unit is configured to compute for each of the clusters of a clustering result of the first sample, a representative value of the second data of cells of the first sample belonging to each cluster and map on the basis of second data of the second sample each of the cells in the second sample onto the cluster with the shortest distance among clusters of the clustering result of the first sample;
wherein
the sample comparison unit compares the clustering result of the first sample and the mapping result of the second sample, and thereby specifies a cluster in which an amount of change between the first sample and the second sample is a threshold value or greater, and
the output unit additionally outputs at least one or more of the first data and the second data about the cells included in the specified cluster.

## Patentansprüche

1. Informationsverarbeitungseinrichtung (20; 21), umfassend:
eine Informationsspeichereinheit (205), die ein Ergebnis des Erfassens von Licht von Zellen, nämlich erste Daten, und ein Ergebnis des Trennens der ersten Daten in eine Vielzahl von Fluoreszenzen, nämlich zweite Daten, in Zuordnung zueinander speichert;
eine Clustering-Einheit (207), die die Zellen basierend auf den zweiten Daten in eine Vielzahl von Clustern clustert; und
eine Ausgabeeinheit (209), die ein Clustering-Ergebnis von der Clustering-Einheit ausgibt, wobei
die Ausgabeeinheit zusätzlich mindestens eine oder mehrere der ersten Daten und der zweiten Daten über die Zellen ausgibt, die in einem von einem Benutzer aus der Vielzahl von Clustern ausgewählten Cluster eingeschlossen sind;
eine Probenvergleichseinheit (211), die eine erste Probe, die eine Vielzahl von Zellen umfasst, für die die ersten Daten und die zweiten Daten in der Informationsspeichereinheit gespeichert sind, und eine zweite Probe, die eine Vielzahl von Zellen umfasst, miteinander vergleicht;
wobei
die Probenvergleichseinheit (211) konfiguriert ist, um für jeden der Cluster eines Clustering-Ergebnisses der ersten Probe einen repräsentativen Wert der zweiten Daten von Zellen der ersten Probe, die zu jedem Cluster gehören, zu berechnen und basierend auf den zweiten Daten der zweiten Probe jede der Zellen in der zweiten Probe auf den Cluster mit dem kürzesten Abstand unter den Clustern des Clustering-Ergebnisses der ersten Probe abzubilden;
wobei
die Probenvergleichseinheit das Clustering-Ergebnis der ersten Probe und das Abbildungsergebnis der zweiten Probe vergleicht und dadurch ein Cluster spezifiziert, in dem ein Betrag der Änderung zwischen der ersten Probe und der zweiten Probe ein Schwellenwert oder größer ist, und
die Ausgabeeinheit zusätzlich mindestens eine oder mehrere der ersten Daten und der zweiten Daten über die Zellen ausgibt, die in dem spezifizierten Cluster eingeschlossen sind.

2. Informationsverarbeitungseinrichtung nach Anspruch 1, wobei
eine Dimensionalität der ersten Daten größer ist als eine Dimensionalität der zweiten Daten.

3. Informationsverarbeitungseinrichtung nach Anspruch 1, wobei
die ersten Daten optische Spektraldaten von Licht aus den Zellen sind.

4. Informationsverarbeitungseinrichtung nach Anspruch 1, wobei
die zweiten Daten als eine Kombination von Fluoreszenzen ausgegeben werden, die aus der Vielzahl der Fluoreszenzen ausgewählt sind.

5. Informationsverarbeitungseinrichtung nach Anspruch 1, wobei
das Clustering-Ergebnis als Bildanzeige ausgegeben wird.

6. Informationsverarbeitungseinrichtung nach Anspruch 1, ferner umfassend:
eine Zellenabfrageeinheit, die Informationen zu den Zellen, die in dem von der Probenvergleichseinheit spezifizierten Cluster eingeschlossen sind, in eine Datenbank zur Spezifizierung der Zellen eingibt.

7. Informationsverarbeitungseinrichtung nach Anspruch 6, wobei
die Zellenabfrageeinheit einen Zelltyp der in dem Cluster eingeschlossenen Zellen basierend auf einem Ergebnis der Abfrage der Datenbank spezifiziert, und
die Ausgabeeinheit zusätzlich mindestens eine oder mehrere der ersten Daten und der zweiten Daten über die Zellen mit dem spezifizierten Zelltyp ausgibt.

8. Informationsverarbeitungseinrichtung nach Anspruch 6, wobei
die Informationen zu den Zellen, die in die Datenbank eingegeben werden, basierend auf den zweiten Daten erzeugt werden.

9. Informationsverarbeitungsverfahren, umfassend:
Speichern eines Ergebnisses des Erfassens von Licht von Zellen, nämlich erste Daten, und ein Ergebnis des Trennens der ersten Daten in eine Vielzahl von Fluoreszenzen, nämlich zweite Daten, in Zuordnung zueinander;
Clustern der Zellen basierend auf den zweiten Daten in eine Vielzahl von Clustern; und
Ausgeben eines Clustering-Ergebnisses von der Clustering-Einheit; und
zusätzlich Ausgeben mindestens einer oder mehrerer der ersten Daten und der zweiten Daten über die Zellen, die in einem von einem Benutzer aus der Vielzahl von Clustern ausgewählten Cluster eingeschlossen sind;
Vergleichen einer ersten Probe, die eine Vielzahl von Zellen umfasst, für die die ersten Daten und die zweiten Daten in der Informationsspeichereinheit gespeichert sind, und einer zweiten Probe, die eine Vielzahl von Zellen umfasst, miteinander;
wobei das Vergleichen das Berechnen eines repräsentativen Wertes der zweiten Daten der Zellen der ersten Probe, die zu jedem Cluster gehören, für jeden der Cluster eines Clustering-Ergebnisses der ersten Probe und das Abbilden jeder der Zellen in der zweiten Probe basierend auf den zweiten Daten der zweiten Probe auf den Cluster mit dem kürzesten Abstand unter den Clustern des Clustering-Ergebnisses der ersten Probe umfasst;
wobei
das Verfahren ferner das Vergleichen des Clustering-Ergebnisses der ersten Probe und des Abbildungsergebnisses der zweiten Probe umfasst, und dadurch ein Cluster spezifiziert, in dem ein Betrag der Änderung zwischen der ersten Probe und der zweiten Probe ein Schwellenwert oder größer ist, und
Ausgeben von mindestens einem oder mehreren der ersten Daten und der zweiten Daten über die Zellen, die in dem spezifizierten Cluster eingeschlossen sind.

10. Programm, das einen Computer veranlasst, zu funktionieren als:
eine Informationsspeichereinheit (205), die ein Ergebnis des Erfassens von Licht von Zellen, nämlich erste Daten, und ein Ergebnis des Trennens der ersten Daten in eine Vielzahl von Fluoreszenzen, nämlich zweite Daten, in Zuordnung zueinander speichert;
eine Clustering-Einheit (207), die die Zellen basierend auf den zweiten Daten in eine Vielzahl von Clustern clustert; und
eine Ausgabeeinheit (209), die ein Clustering-Ergebnis von der Clustering-Einheit ausgibt, wobei
die Ausgabeeinheit so ausgelegt ist, dass sie zusätzlich mindestens eine oder mehrere der ersten Daten und der zweiten Daten über die Zellen ausgibt, die in einem von einem Benutzer aus der Vielzahl von Clustern ausgewählten Cluster eingeschlossen sind;
eine Probenvergleichseinheit (211), die eine erste Probe, die eine Vielzahl von Zellen umfasst, für die die ersten Daten und die zweiten Daten in der Informationsspeichereinheit gespeichert sind, und eine zweite Probe, die eine Vielzahl von Zellen umfasst, miteinander vergleicht;
wobei
die Probenvergleichseinheit konfiguriert ist, um für jeden der Cluster eines Clustering-Ergebnisses der ersten Probe einen repräsentativen Wert der zweiten Daten von Zellen der ersten Probe, die zu jedem Cluster gehören, zu berechnen und basierend auf den zweiten Daten der zweiten Probe jede der Zellen in der zweiten Probe auf den Cluster mit dem kürzesten Abstand unter den Clustern des Clustering-Ergebnisses der ersten Probe abzubilden;
wobei
die Probenvergleichseinheit das Clustering-Ergebnis der ersten Probe und das Abbildungsergebnis der zweiten Probe vergleicht und dadurch ein Cluster spezifiziert, in dem ein Betrag der Änderung zwischen der ersten Probe und der zweiten Probe ein Schwellenwert oder größer ist
und
die Ausgabeeinheit zusätzlich mindestens eine oder mehrere der ersten Daten und der zweiten Daten über die Zellen ausgibt, die in dem spezifizierten Cluster eingeschlossen sind.

## Revendications

1. Appareil de traitement d'informations (20, 21) comprenant :
une unité de stockage d'informations (205) qui stocke un résultat de détection de la lumière provenant de cellules, à savoir des premières données, et un résultat de séparation des premières données en une pluralité de fluorescences, à savoir des secondes données, en association les unes avec les autres ;
une unité de regroupement (207) qui regroupe les cellules en une pluralité de groupes sur une base des secondes données ; et
une unité de sortie (209) qui émet un résultat de regroupement à partir de l'unité de regroupement, dans lequel
l'unité de sortie émet en plus au moins une ou plusieurs des premières données et des secondes données concernant les cellules incluses dans un groupe sélectionné par un utilisateur parmi la pluralité de groupes ;
une unité de comparaison d'échantillons (211) qui compare un premier échantillon comprenant une pluralité de cellules, pour lequel les premières données et les secondes données sont stockées dans l'unité de stockage d'informations, et un second échantillon comprenant une pluralité de cellules les unes par rapport aux autres ;
dans lequel
l'unité de comparaison d'échantillons (211) est configurée pour calculer, pour chacun des groupes d'un résultat de regroupement du premier échantillon, une valeur représentative des secondes données des cellules du premier échantillon appartenant à chaque groupe et mapper sur la base des secondes données du second échantillon chacune des cellules du second échantillon sur le groupe avec la distance la plus courte parmi les groupes du résultat de regroupement du premier échantillon ;
dans lequel
l'unité de comparaison d'échantillons compare le résultat de regroupement du premier échantillon et le résultat de mappage du second échantillon, et spécifie ainsi un groupe dans lequel une quantité de changement entre le premier échantillon et le second échantillon est une valeur seuil ou supérieure, et
l'unité de sortie émet en plus au moins une ou plusieurs des premières données et des secondes données concernant les cellules incluses dans le groupe spécifié.

2. Appareil de traitement d'informations selon la revendication 1, dans lequel
une dimensionnalité des premières données est supérieure à la dimensionnalité des secondes données.

3. Appareil de traitement d'informations selon la revendication 1, dans lequel
les premières données sont des données de spectre optique de la lumière provenant des cellules.

4. Appareil de traitement d'informations selon la revendication 1, dans lequel
les secondes données sont émises comme combinaison de fluorescences choisies parmi la pluralité de fluorescences.

5. Appareil de traitement d'informations selon la revendication 1, dans lequel le résultat de regroupement est émis comme affichage d'image.

6. Appareil de traitement d'informations selon la revendication 1, comprenant en outre :
une unité d'interrogation de cellule qui entre des informations relatives aux cellules incluses dans le groupe spécifié par l'unité de comparaison d'échantillons dans une base de données permettant de spécifier les cellules.

7. Appareil de traitement d'informations selon la revendication 6, dans lequel
l'unité d'interrogation de cellule spécifie un type de cellule des cellules incluses dans le groupe sur une base d'un résultat d'interrogation dans la base de données, et
l'unité de sortie émet en plus au moins une ou plusieurs des premières données et des secondes données concernant les cellules avec le type de cellule spécifié.

8. Appareil de traitement d'informations selon la revendication 6, dans lequel
les informations relatives aux cellules qui sont entrées dans la base de données sont générées sur la base des secondes données.

9. Procédé de traitement d'informations comprenant :
le stockage d'un résultat de détection de la lumière provenant de cellules, à savoir des premières données, et un résultat de séparation des premières données en une pluralité de fluorescences, à savoir des secondes données, en association les unes avec les autres ;
le regroupement des cellules en une pluralité de groupes sur une base des secondes données ; et
l'émission d'un résultat de regroupement à partir de l'unité de regroupement ; et
l'émission en plus d'au moins une ou plusieurs des premières données et des secondes données concernant les cellules incluses dans un groupe sélectionné par un utilisateur parmi la pluralité de groupes ;
la comparaison d'un premier échantillon comprenant une pluralité de cellules, pour lequel les premières données et les secondes données sont stockées dans l'unité de stockage d'informations, et d'un second échantillon comprenant une pluralité de cellules les unes par rapport aux autres ;
dans lequel la comparaison comprend le calcul, pour chacun des groupes d'un résultat de regroupement du premier échantillon, d'une valeur représentative des secondes données des cellules du premier échantillon appartenant à chaque groupe et le mappage, sur la base des secondes données du second échantillon, de chacune des cellules dans le second échantillon sur le groupe avec la distance la plus courte parmi des groupes du résultat de regroupement du premier échantillon ;
dans lequel
le procédé comprend en outre la comparaison du résultat de regroupement du premier échantillon et du résultat de mappage du second échantillon, et spécifie ainsi un groupe dans lequel une quantité de changement entre le premier échantillon et le second échantillon est une valeur seuil ou supérieure, et
l'émission d'au moins une ou plusieurs des premières données et des secondes données concernant les cellules incluses dans le groupe spécifié.

10. Programme amenant un ordinateur à fonctionner en tant que :
une unité de stockage d'informations (205) qui stocke un résultat de la détection de la lumière provenant de cellules, à savoir des premières données, et un résultat de la séparation des premières données en une pluralité de fluorescences, à savoir des secondes données, en association les unes avec les autres ;
une unité de regroupement (207) qui regroupe les cellules en une pluralité de groupes sur une base des secondes données ; et
une unité de sortie (209) qui émet un résultat de regroupement à partir de l'unité de regroupement, dans lequel
l'unité de sortie est mise en fonctionnement pour produire en plus au moins une ou plusieurs des premières données et des secondes données concernant les cellules incluses dans un groupe sélectionné par un utilisateur parmi la pluralité de groupes ;
une unité de comparaison d'échantillons (211) qui compare un premier échantillon comprenant une pluralité de cellules, pour lequel les premières données et les secondes données sont stockées dans l'unité de stockage d'informations, et un second échantillon comprenant une pluralité de cellules les unes par rapport aux autres ;
dans lequel
l'unité de comparaison d'échantillons est configurée pour calculer, pour chacun des groupes d'un résultat de regroupement du premier échantillon, une valeur représentative des secondes données des cellules du premier échantillon appartenant à chaque groupe, et mapper sur la base des secondes données du second échantillon chacune des cellules dans le second échantillon sur le groupe avec la distance la plus courte parmi les groupes du résultat de regroupement du premier échantillon ;
dans lequel
l'unité de comparaison d'échantillons compare le résultat de regroupement du premier échantillon et le résultat de mappage du second échantillon, et spécifie ainsi un groupe dans lequel une quantité de changement entre le premier échantillon et le second échantillon est une valeur seuil ou supérieure,
et
l'unité de sortie émet en plus au moins une ou plusieurs des premières données et des secondes données concernant les cellules incluses dans le groupe spécifié.
